(19) Europäisches Patentamt
European Patent Office
Office européen des brevets

(11) Publication number: 0 000 272
B1

(12) **EUROPEAN PATENT SPECIFICATION**

(45) Date of publication of patent specification: 05.05.82

(21) Application number: 78300076.3

(22) Date of filing: 23.06.78

(51) Int. Cl.³: C 07 D 501/36,
A 61 K 31/545,
C 07 D 257/04

# (54) 7-Acylamino-3-substituted-3-cephem-4-carboxylic acids, processes for their preparation and pharmaceutical compositions containing them.

(30) Priority: 24.06.77 US 809585

(43) Date of publication of application:
10.01.79 Bulletin 79/1

(45) Publication of the grant of the patent:
05.05.82 Bulletin 82/18

(84) Designated Contracting States:
BE CH DE FR GB LU NL SE

(56) References cited:
FR - A - 2 255 077

(73) Proprietor: SMITHKLINE CORPORATION
1500 Spring Garden Street P.O. Box 7929
Philadelphia Pennsylvania 19101 (US)

(72) Inventor: Berges, Davis Alan
18, Buckwalter Road
Phoenixville Pennsylvania 19460 (US)

(74) Representative: Hargreaves, Gerald Henry, Dr. et al,
P.O.Box 39
Welwyn Garden City Hertfordshire AL7 1EY (GB)

EP 0 000 272 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

7-Acylamino-3-substituted-3-cephem-4-carboxylic acids, processes for their preparation and pharmaceutical compositions containing them

This invention relates to cephalosporin derivatives having antibacterial activity their preparation and pharmaceutical compositions containing them.

According to the present invention there is provided a compound of formula (I)

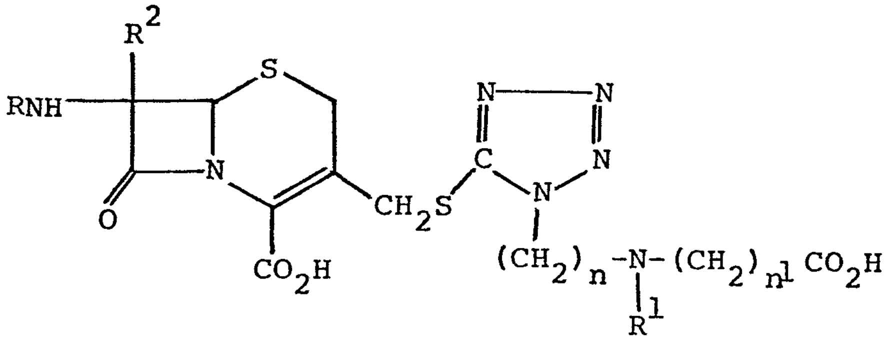

(I)

and pharmaceutically acceptable salts, esters, easily split ethers and amide derivatives thereof where R is

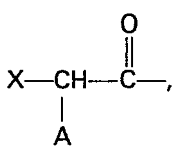, 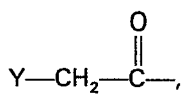, or 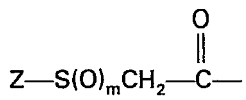

X is thienyl, furyl, phenyl, or phenyl monosubstituted with hydroxy, hydroxymethyl, formamido or ureido;

A is $NH_2$ OH, COOH, $SO_3H$, formyloxy or, when the $\alpha$-C-hydrogen is absent, methoxyimino;

Y is cyano, sydnonyl, pyridonyl, thienyl, *o*-aminomethylphenyl, phenyl or tetrazolyl;

Z is methyl, trifluoromethyl, trifluoroethyl, pyridyl or cyanomethyl;

$R^1$ is hydrogen or a lower alkyl group having from one to four carbon atoms or $R^2$ is hydrogen or methoxy;

m is from zero to two;

n is two to four and $n^1$ is one to four.

Each of the three partial structures above represent subgeneric groups of compounds covered by this invention.

Examples of the substituent RHN— are:—

$\alpha$-hydroxyphenylacetamido
$\alpha$-aminophenylacetamido
$\alpha$-amino-4-hydroxyphenylacetamido
trifluromethylthioacetamido
2,2,2-trifluoroethylsulfinylacetamido
2,2,2-trifluoroethylthioacetamido
cyanoacetamido
$\alpha$-carboxythienylacetamido
$\alpha$-carboxyphenylacetamido
$\alpha$-sulphophenylacetamido
methylsulfonylacetamido
cyanomethylthioacetamido
3-sydnoneacetamido
1-tetrazolylacetamido
2-thienylacetamido
$\alpha$ (Z)-(methoxyimino)-2-furanacetamido
*o*-aminomethylphenylacetamido and
4-pyridylthioacetamido.

When the substituent R is asymetric for example when R is mandeloyl or phenylglycyl optical isomers are possible and the D-forms are preferred.

It will be recognised that the cephem-4-carboxlic acid group, the carboxylic acid group of the tetrazole substituent and the group A when it is —COOH, can form esters and salts. Accordingly by a

pharmaceutical ester or salt of a compound of formula (I) is meant an ester or salt of one or more of these carboxylic acid groups.

Examples of such esters include simple alkyl and aryl esters and in particular esters which are easily cleared within the body to the parent acid in particular indanyl, pivaloyloxymethyl, acetoxymethyl, propionyloxymethyl, glycyloxymethyl, phenylglycyloxymethyl and thienylglycyloxymethyl esters.

Examples of salts of compounds of formula (I) include alkali metal salts for example the sodium and potassium salts, the ammonium salt and salts with organic amines for example procaine and dibenzylethylene diamine.

Hydroxyl groups in the substituent R can form easily split esters or ethers.

Amino groups in the substituent R for example when RNH— is 7-phenylglycylamino can be converted into pharmaceutically acceptable amide derivatives for example furanyl-, pyranyl-, oxolanyl- or oxiranylcarbonyl amides (see Belgian Patent No 835, 295).

Compounds of the invention may exist as solvates for examples hydrates, glycolates and alcoholates. Such forms are of course within the scope of this invention.

Compounds of formula (I) can be prepared by reacting compound of formula (II):—

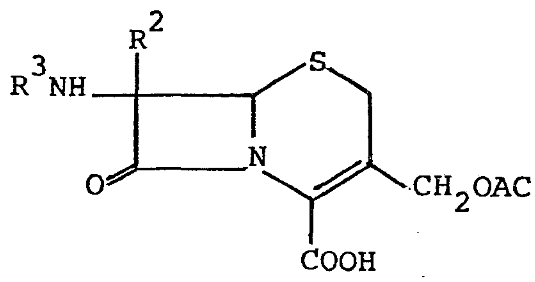

(II)

where AC is acetyl or a derivative thereof where the cephem-4-carboxylic acid group is protected where $R^3$ is hydrogen or a group R as previously defined provided that any amino, carboxy, sulfo, or hydroxy groups are optionally protected, with a compound of formula (III):—

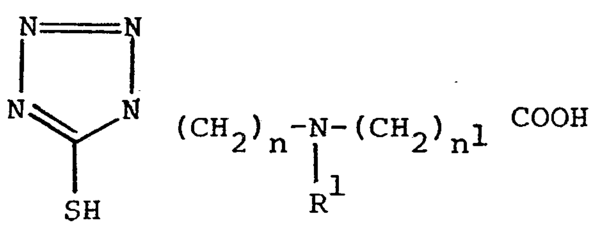

(III)

where $R^1$, n and $n^1$ are as defined with reference to formula I or an alkali metal salt thereof and where $R^3$ is hydrogen thereafter reacting the product with an acylating agent or activated derivative of an acid ROH where R is as defined with reference to formula (I) provided that any free amino, carboxy, hydrogen or sulfo groups are optionally protected thereafter where $R^1$ is hydrogen optionally acylating the product with an N-acylating agent which supplies lower alkanoyl group thereafter removing any protecting groups and optionally converting the compound of formula (I) so obtained into a salt, ester, easily split ether or amide derivative thereof.

The compounds of formula (I) are most conveniently prepared by displacement of acetoxy from a compound of formula (II) above where $R^3$ is a group R with a compound of formula (III) above.

Compounds of formula (II) are known or can be prepared by known methods.

The acylation steps referred to in the above process can be carried out by known methods for example as disclosed in Cephalosporins and Penicillins, Flynn, Academic Press, 1972; US Patent Nos. 2,721,196 and 3,953,424; Belgian Patent No. 832,725; German Patent Nos. 2,127,285 and 2,406,165.

Protecting groups which can be used during the process described above are known (see "Protective Groups in Organic Chemistry" J.F.W. McOmie, Plenum Press, 1973, Chapters 2 and 3 for use of amino, carboxy, sulfo or hydroxy protecting groups). Examples of such groups include *t*-butyl for —COOH and *t*-butoxycarbonyl for —$NH_2$. These particular groups can be removed easily using trifluoroacetic acid.

Pharmaceutically acceptable salts, esters, easily split ethers and amide derivatives of compounds of formula (I) can be prepared by known methods, in particular sodium or potassium salts can be prepared from sodium or potassium 2-ethylhexanoate.

Tetrazole-5-thiones of formula (III) are disclosed and claimed in our co-pending European Patent Application No. 80200260.0.

The compounds of formula (I) have antibacterial activity against both Gram positive and Gram negative bacteria with minimum inhibitory concentrations (MIC's) *in vitro* from 0.2 to 200 μg/ml. Test results for 7 - D - mandel - amido - 3 - [1 - [2 - (carboxymethylamino)ethyl]ethyl]tetrazol - 5 - ylthiomethyl] - 3 - cephem; 4 - carboxylic acid hydrate (A) are:

| | A | Cefazolin | Cephalothin |
|---|---|---|---|
| *S. aureus HH 127* | 3.1 | 0.4 | 0.2 |
| *S. aureus SK 23390* | 1.6 | 0.2 | ≤0.1 |
| *S. aureus villaluz* SK 70390 | 100 | 100 | 50 |
| *Strep. faecalis* HH 34358 | 50 | 6.3 | 12.5 |
| *E. coli* SK 12140 | 0.8 | 0.8 | 3.1 |
| *E. coli* HH 33779 | 0.8 | 0.8 | 6.3 |
| *Kleb. pneumo.* SK 4200 | 0.4 | 0.8 | 1.6 |
| *Kleb. pneumo.* SK 1200 | 0.4 | 0.8 | 1.6 |
| *Salmonella ATCC* 12176 | 0.2 | 0.8 | 0.8 |
| *Pseudo. aeru.* HH 63 | ≥200 | ≥200 | ≥200 |
| *Serratia marc.* ATCC 13880 | 3.1 | 200 | ≥200 |
| *Proteus morgani* 179 | 3.1 | 200 | 200 |
| *Entero. aerog.* ATCC 13048 | 1.6 | 1.6 | 12.5 |
| *Entero. cloacae* NH 31254 | 0.8 | 0.8 | 6.3 |
| *Proteus mirabilis* PN—444 | 0.8 | 3.1 | 6.3 |

Compound A gave an $ED_{50}$ in mice of 0.39 mg/kg (s.c.) and 7.2 mg/kg (p.o.) against E. coli, and 0.39 mg/kg against Kleb. pneumo. (s.c.) and 4 mg/kg (p.o.). Cephalexin gives comparable values of 15.7 (s.c.) and 25 (p.o.) against E. Coli and 21.5 mg/kg (s.c.) and 18 mg/kg (p.o.) against Kleb. pneumo.

The invention further provides a pharmaceutical composition comprising a compound of the invention and a pharmaceutically acceptable carrier.

The compositions of the invention can be formulated so that they can be administered orally or by parenteral injection for example intravenously or intramuscularly.

Preferably the composition is in the form of an injectable sterile solution or suspension.

The compounds of the invention can be formulated in the same way as known cephalosporins for example cefazolin or cephalothin.

In use in the treatment of bacterial infections in man and animals effective but non-toxic amount of the compound of the invention is administered in unit doses of preferably from 250 mg to 600 mg calculated as compound of formula (I) with a total daily dosage of from 750 mg to 6 gm.

The precise dosages are dependent upon the age and weight of the subject and on the susceptibility of the infection being treated. These can be determined by those skilled in the art based on the data disclosed herein compared with that available in the art attained with known cephalosporins.

The following examples illustrate the invention:—

Temperatures are in degrees Centigrade (°C.) unless otherwise stated.

Example 1

A mixture of 21.5 g. (11.4 mmol) of 1 - [2 - (acetylamino)ethyl] - 1,4 - dihydro - 5*H* - tetrazole - 5 - thione in 300 ml. of 6N hydrochloric acid was heated at reflux for 3.5 hours. The mixture was filtered after cooling to room temperature. The filtrate was concentrated to small volume. The residual liquid was diluted with i-propanol. The solid which precipitated was filtered, washed and dried *in vacuo* to give 13.7 g. of 1 - (2 - aminoethyl) - 1,4 - dihydro - 5*H* - tetrazole - 5 - thione, hydrochloride (66.1% yield) mp 232—233.5°C.

To a solution of 22.8 g. (12.5 mmol) of 1 - (2 - aminoethyl) - 1,4 - dihydro - 5*H* - tetrazole -

5 - thione, hydrochloride in 100 ml. of N,N-dimethylformamide and 100 ml. of acetone was added 34.3 ml. (25 mmol) of triethylamine. To the resulting suspension was added slowly a solution of 19.5 g. (12.5 mmol) of *p*-methoxybenzyl chloride in 30 ml. of acetone. After stirring at room temperature for 15 hours, the mixture was filtered. The filtrate was evaporated to dryness. The residue was taken up in 350 ml. of 5% $NaHCO_3$, and extracted with ethyl acetate. The combined extract was dried ($MgSO_4$) and evaporated to dryness to give an oil which was chromatographed on a silica gel column, eluting with a gradient of 5—10% ethanol in chloroform. Fractions containing product by thin layer chromatography were pooled, and evaporated to dryness to give 1 - (2 - aminoethyl) - 5 - (4 - methoxybenzylthio) - 1*H* - tetrazole as a brown oil (26 g., 80%). An analytical sample of the crystalline amine hydrochloride (m. 148—150°) was obtained by treating the product with an ethereal HCl solution.

To a solution of 15.0 g. (56 mmol) of 1 - (2 - aminoethyl) - 5 - [(4 - methoxybenzyl)thiol - 1*H* - tetrazole in 70 ml. of dry tetrahydrofuran was added 7.7 ml. (56 mmol) of triethylamine, and 6.2 ml. (56 mmol) of ethyl bromoacetate. After stirring at room temperature for 15 hours, the mixture was filtered, and the filtrate was evaporated *in vacuo* to dryness. The residue was dissolved in 70 ml. of chloroform and decolorized with charcoal. The filtrate was chromatographed on silica gel, eluting with a gradient of 0—15% ethyl acetate in chloroform. Fractions containing product by thin layer chromatography were pooled and evaporated to dryness to give 12.5 g. (62% yield) of 1 - [2 - [[(carbethoxy)-methyl]amino]ethyl] - 5 - [(4 - methoxybenzyl)thio] - 1*H* - tetrazole as a brown oil.

To a solution of 12.5 g. (35.6 mmol) 1 - [2 - [[(carbethoxy)methyl]amino]ethyl] - 5 - [(4 - methoxybenzyl)thio] - 1*H* - tetrazole in 250 ml. of methanol and 65 ml. of water was added a solution of 25.5 g. (80 mmol) of mercuric acetate in 80 ml. of water. The mixture was stirred at room temperature for 15 hours and at reflux for 1 hour. After thorough cooling, the mixture was treated with hydrogen sulfide gas for 1.5 hours. The dark mixture was heated over a steam bath for 1.5 hours and filtered. The filtrate was evaporated *in vacuo* to dryness. The residue was recrystallized from ethyl acetate to give 5.9 g. of 1 - [2 - [(carboxymethyl)amino]ethyl] - 1,4 - dihydro - 5*H* - tetrazole - 5 - thione (82.0% yield) mp 215—220° dec.

To a solution of 420 mg. (5 mmol) of sodium bicarbonate in 25 ml. of water was added 1.01 g. (5 mmol) of 1 - [2 - [(carboxymethyl)amino]ethyl] - 1,4 - dihydro - 5*H* - tetrazole - 5 - thione. After $CO_2$ gas evolution had ceased, 2.6 g. (6 mmol) of 7-D-mandelamidocephalosporanic acid, sodium salt, was added to the solution. The mixture was stirred and heated at 65°C., while pH was maintained at 7.0 by addition of a 5% $NaHCO_3$ solution. After 2 hours the mixture was filtered. The filtrate was applied to a Biogel P—2 (100—200 U.S. standard mesh) column, eluting with de-ionized water. Fractions containing product by thin layer chromatography were pooled, concentrated to small volume, and applied to a cellulose column. A mixture of acetonitrile and water (8 to 2) was used as chromatographic solvent. The eluate that contained product was evaporated to dryness. The residue was dissolved in deionized water and solution was lyophilized to give 290 mg. of 7 - D - mandelamido - 3 - [1 - [2 - [(carboxymethyl)amino]ethyl]tetrazole - 5 - ylthiomethyl] - 3 - cephem - 4 - carboxylic acid (10% yield) mp 170—173°C. decomp.

## Example 2

Substituting in the above procedure equimolar quantities of 1 - [3 - (acetylamino)propyl] - 1,4 - dihydro - 5*H* - tetrazole - 5 - thione or 1 - [4 - (acetylamino)butyl] - 1,4 - dihydro - 5*H* - tetrazole - 5 - thione (prepared as described in the art from N-(3-aminopropyl)acetamide and N-(4-aminobutyl)acetamide respectively gives 7 - (D - mandelamido) - 3 - [1 - [3 - [(carboxymethyl)-amino]propyl]tetrazole - 5 - ylthiomethyl] - 3 - cephem - 4 - carboxylic acid and 7 - (D - mandelamido) - 3 - [1 - [4[(carboxymethyl)amino]butyl]tetrazol - 5 - ylthiomethyl] - 3 - cephem - 4 - carboxylic acid. Substituting ethyl 4 - bromobutyrate in place of ethyl bromoacetate above gives 1 - [2 - [(B - carboxypropyl)amino]ethyl] - 1,4 - dihydro - 5H - tetrazole - 5 - thione and 7 - (D - mandelamido) - 3 - [1 - [2 - [(3 - carboxypropyl)amino]ethyl] - 1H - tetrazol - 5 - ylthio-methyl] - 3 - cephem - 4 - carboxylic acid.

## Example 3

A mixture of 5.22 g. (10.0 mmol) of 7 - [D - $\alpha$ - (*t* - butoxycarbonyl)amino - $\alpha$ - (4 - hydroxy-phenyl)acetamido]cephalosporanic acid and an excess (15.0 mmol) of 1 - [2 - [(carboxymethyl)-amino]ethyl] - 1,4 - dihydro - 5*H* - tetrazole - 5 - thione in 75 ml. of water is treated with sufficient sodium bicarbonate to give a solution of pH 7.0. The solution is heated at 70° for 3 hours, cooled, and added to a XAD-7 resin column. Elution with water and then methanol followed by evaporation of the product-containing fractions gives the *t*-boc derivative of the desired compound. This derivative is stirred at 25°C. with 25 ml. of trifluoroacetic acid and 25 ml. of 1,3-dimethoxybenzene for 2 hours. The mixture is evaporated to dryness, either added to the residue and the precipitated salt collected. This is dissolved in water and two molecular equivalents of sodium bicarbonate are added. The solution is lyophilized and then triturated with acetone to give 7 - [D - $\alpha$ - amino - $\alpha$ - (4 - hydroxyphenyl)-acetamido] - 3 - [1 - [2 - [(carboxymethyl)amino]ethyl]tetrazol - 5 - ylthiomethyl] - 3 - cephem - 4 - carboxylic acid. Similar treatment of the *t*-boc derivative of the 7 - D - ($\alpha$ - amino - $\alpha$ - phenyl-acetamido)cephalosporanic acid gives the corresponding 7 - D - ($\alpha$ - amino - $\alpha$ - phenyl-

acetamido) - 3 - [1 - [2 - [(carboxymethyl)amino]ethyl]tetrazol - 5 - ylthiomethyl] - 3 - cephem - 4 - carboxylic acid.

Example 4

A mixture of an excess (12.2 mmol) of 1 - [2 - [(carboxymethyl)amino]ethyl] - 1,4 - dihydro - 5*H* - tetrazole - 5 - thione, 32.5 mmol of sodium bicarbonate and 8.1 mmol of 7 - trifluoromethylthioacetamidocephalosporanic acid in 50 ml. of water is stirred at 70° for 5 hours. The reaction mixture is cooled and passed over XAD-2 resin with water and methanol as eluants. The product-containing fractions are evaporated to dryness to give a residue which is dissolved in a small amount of water and lyophilized to give 7 - trifluoromethylthioacetamido - 3 - [1 - [2 - [(carboxymethyl-amino]ethyl]tetrazol - 5 - ylthiomethyl] - 3 - cephem - 4 - carboxylic acid disodium salt. Substituting 7 - (2 - thienylacetamido)cephalosporanic acid gives 7 - (2 - thienylacetamido) - 3 - [1 - [2 - [(carboxymethyl)amino]ethyl]tetrazol - 5 - ylthiomethyl] - 3 - cephem - 4 - carboxylic acid disodium salt.

Stoichiometric quantities of cephalosporanic acids having the individual 7-acylamino substituent listed hereabove may be substituted in Examples 1—3 with variations which will be obvious to those skilled in this art.

Example 5

To a solution of 10 mmol of 1 - [2 - [[(carbethoxy)methyl]amino]ethyl] - 5 - [(4 - methoxybenzyl)thio] - 1*H* - tetrazole and 10 mmol of triethylamine in 20 ml. of dry tetrahydrofuran is added 10 mmol of methyliodide. After stirring at room temperature for 24 hours, the mixture is filtered. The filtrate is stripped *in vacuo* to dryness, and the residue is dissolved in chloroform and chromatographed on silica gel eluting with a gradient of ethylacetate in chloroform. Evaporation of the product-containing fractions gives 1 - [2 - [[(carbethoxy)methyl]methylamino]ethyl - 5 - [(4 - methoxylbenzyl)thio] - 1*H* - tetrazole. Deblocking with mercuric acetate as above gives 1 - [2 - [(carboxymethyl)methyl-amino]ethyl] - 1,4 - dihydro - 5*H* - tetrazole - 5 - thione which when substituted in the reaction with 7 - D - mandelamidocephalosporanic acid gives 7 - D - mandelamido - 3 - [1 - [2 - [(carboxymethyl)methylamino]ethyl]tetrazol - 5 - ylthiomethyl] - 3 - cephem - 4 - carboxylic acid.

Example 6

An injectable pharmaceutical composition is formed by adding sterile saline solution (2 ml.) to 500 mg. of the product of Example 1. This material is injected parenterally four times daily to a human patient infected with susceptible bacteria. Other compounds of this invention may be similarly used.

Example 7

An aqueous solution of 4.27 g. (0.0096 mmol) of 7 - [$\alpha$(Z) - (methoxyimino) - 2 - furanacetamido]cephalosporanic acid sodium salt, 1.78 g. (0.0212 mmol) of sodium bicarbonate, and 2.15 g. (.0106 mmol) of 1 - [2 - [(carboxymethyl)amino]ethyl] - 1,4 - dihydro - 5*H* - tetrazole - 5 - thione is heated at 65°C. for 6 hours during which time the pH is maintained at 7.6—7.8 with dilute sodium bicarbonate. After cooling, the reaction mixture is purified on an XAD-2 column as described in Example 4 to give a lyophilized product, 7 - [$\alpha$(Z) - (methoxyimino) - 2 - furanacetamido] - 3 - [1 - [2 - [(carboxymethyl)amino]ethyl] - tetrazole - 5 - ylthiomethyl] - 3 - cephem - 4 - carboxylic acid, disodium salt.

**Claims**

1. A compound of formula (I):—

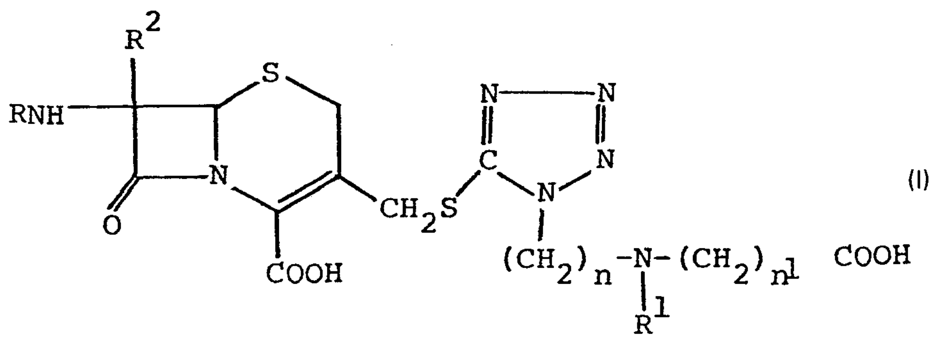

(I)

and pharmaceutically acceptable salts, esters, easily split ethers and amide derivatives thereof where R is:—

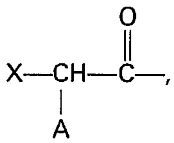, 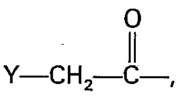, or 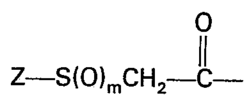

where X is thienyl, furyl, phenyl, or phenyl monosubstituted with hydroxy, hydroxymethyl, formamido or ureido;

A is $NH_2$ OH, COOH, $SO_3H$, formyloxy or, when the $\alpha$-C-hydrogen is absent, methoxyimino;

Y is cyano, sydnonyl, pyridonyl, thienyl, *o*-amino-methylphenyl, phenyl or tetrazolyl;

Z is methyl, trifluoromethyl, trifluoroethyl, pyridyl or cyanomethyl;

$R^1$ is hydrogen or a lower alkyl group having from one to four carbon atoms; $R^2$ is hydrogen or methoxy;

m is from zero to two;

n is two to four and $n^1$ is one to four.

2. The D-form of a compound as claimed in claim 1 having a substituent R which is asymetric.

3. 7 - [$\alpha$(Z) - (methoxyimino) - 2 - furanacetamido] - 3 - [1 - [2 - [(carboxymethyl)-amino]ethyl]tetrazol - 5 - ylthiomethyl] - 3 - cephem - 4 - carboxylic acid.

4. A process for preparing a compound as claimed in claim (1) which comprises reacting a compound of formula (II):—

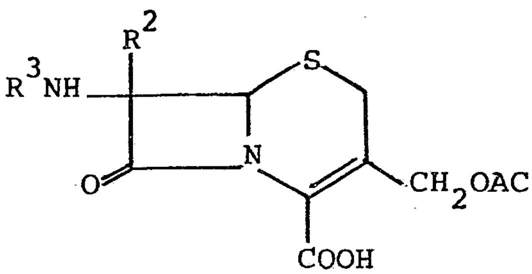

(II)

where AC is acetyl or a derivative thereof where the cephem-4-carboxylic acid group is protected, where $R^3$ is hydrogen or a group R as previously defined provided that any amino, carboxy, sulfo, or hydroxy groups are optionally protected, with a compound of formula (III):—

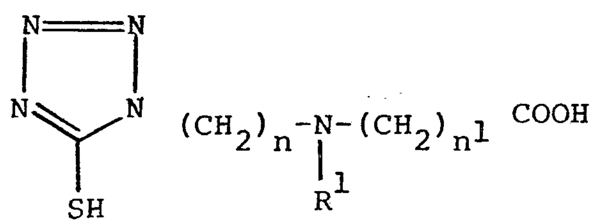

(III)

where $R^1$, n and $n^1$ are as defined with reference to formula I or an alkali metal salt thereof and where $R^3$ is hydrogen thereafter reacting the product with an acylating agent or activated derivative of an acid ROH where R is as defined with reference to formula (I) provided that any free amino, carboxy, hydrogen or sulfo groups are optionally protected thereafter where $R^1$ is hydrogen optionally acylating the product with an N-acylating agent which supplies lower alkanoyl group thereafter removing any protecting groups and optionally converting the compound of formula (I) so obtained into a salt, ester, easily split ether or amide derivative.

5. A pharmaceutical composition comprising a compound as claimed in any one of the claims 1 to 3 and a pharmaceutically acceptable carrier.

**Revendications**

1. Composé de formule (I)

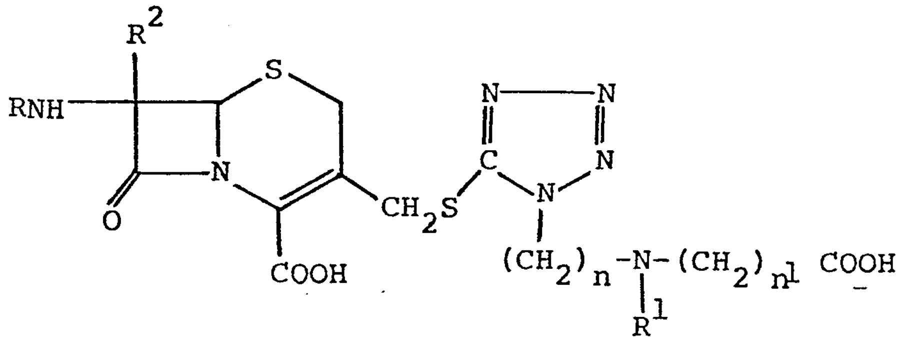

(I)

ainsi que les sels pharmaceutiquement acceptables, les esters et les dérivés éthérés et amidiques aisément clivables de ce composé, dans laquelle R représente:

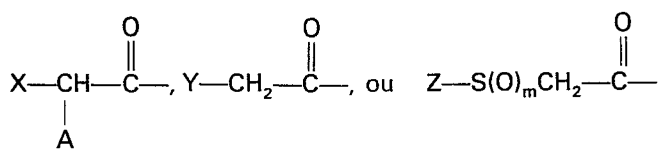

où X représente un groupe thiényle, furyle, phényle, phényle monosubstitué par un groupe hydroxy, hydroxyméthyle, formamido ou uréido;

A représente un groupe $—NH_2$, —OH, —COOH, $—SO_3H$, formyloxy ou méthoxyimino lorsque l'hydrogène sur l'atome de carbone en $\alpha$ est absent;

Y représente un radical cyano, sydnonyle, pyridonyle, thiényle, *o*-aminométhylphényle, phényle ou tétrazolyle;

Z représente un groupe méthyle, trifluorométhyle, trifluoroéthyle, pyridyle ou cyanométhyle;

$R^1$ représente un atome d'hydrogène ou un groupe alcoyle inférieur ayant de un à quatre atomes de carbone;

$R^2$ représente un atome d'hydrogène ou un groupe méthoxy;

m varie de zero à deux;

n varie de deux à quatre; et $n^1$ varie de un à quatre.

2. Forme D d'un composé tel que revendiqué dans la revendication 1, possédant un substituant R qui est asymétrique.

3. Acide 7 - [$\alpha$(Z) - (méthoxyimino) - 2 - furanacétamido] - 3 - [1 - [2 - [(carboxyméthyl)amino]éthyl]tétrazole - 5 - ylthiométhyl] - 3 - céphème - 4 - carboxylique.

4. Procédé de préparation d'un composé suivant la revendication 1 caractérisé en ce qu'on fait réagir un composé de formule (II):

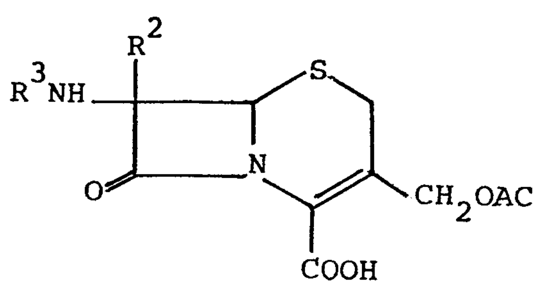

(II)

ou un de ses dérivés dans lequel le groupe acide céphème-4-carboxylique est protégé, où Ac représente un groupe acétyle, $R^3$ représente un atome d'hydrogène ou un groupe R tel que défini précédemment, étant entendu que tout groupe amino, carboxy, sulfo ou hydroxy soit éventuellement protégé, avec un composé de formule (III):

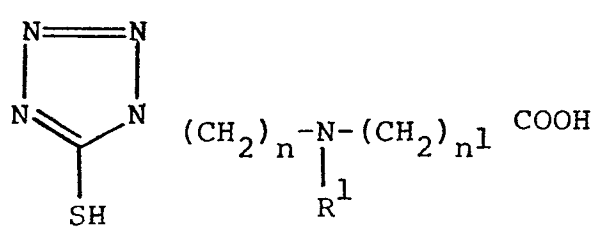

(III)

ou un sel de métal alcalin de ce composé, où $R^1$, n et $n^1$ sont tels que définis dans la formule (I), et, lorsque $R^3$ représente un atome d'hydrogène, on acyle ensuite le produit ainsi obtenu avec un agent acylant ou un dérivé activé d'un acide ROH où R est tel que défini dans la formule (I) étant entendu que tout groupe amino, carboxy, hydroxy ou sulfo libre soit éventuellement protégé et ensuite, lorsque $R^1$ représente un atome d'hydrogène, on acyle éventuellement le produit à l'aide d'un agent N-acylant qui fournit le groupe alcanoyle inférieur, on élimine ensuite tout groupe protecteur et éventuellement, on transforme ensuite le composé de formule (I) ainsi obtenu en un sel pharmaceutiquement acceptable, ester, dérivé éthéré ou amidique aisément scindable.

5. Composition pharmaceutique caractérisée en ce qu'elle contient un composé suivant l'une quelconque des revendications 1 à 3 en association avec un véhicule pharmaceutiquement acceptable.

**Patentansprüche**

1. Eine Verbindung der Formel I

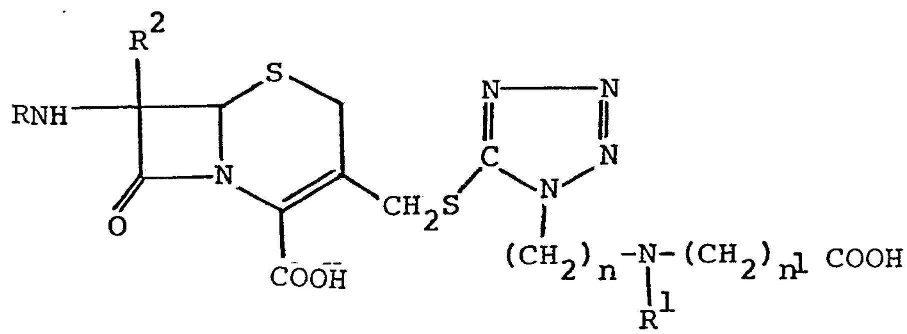

(I)

und ihre pharmazeutisch verträglichen Salze, Ester, leicht zu spaltende Äther und Amidderivate, wobei R die Reste

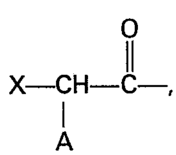 , 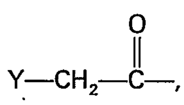 , oder 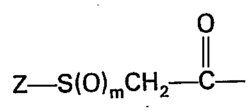

bedeutet, in denen X eine Thienyl- oder Furylgruppe oder eine gegebenenfalls mit einer Hydroxy-, Hydroxymethyl-, Formamido- oder Ureidogruppe einfach substituierte Phenylgruppe ist,

A —$NH_2$, —OH, —COOH, —$SO_3H$, eine Formyloxy, oder bei Abwesenheit eines Wasserstoffatoms ein $\alpha$-Kohlenstoffatom eine Methoxyiminogruppe ist;

Y eine Cyano-, Sydnonyl-, Pyridonyl-, Thienyl-, o-Amino-methylphenyl-, Phenyl- oder Tetrazolylgruppe ist;

Z eine Methyl-, Trifluormethyl-, Trifluoräthyl-, Pyridyl- oder Cyanomethylgruppe ist;

$R^1$ ein Wasserstoffatom oder ein Niederalkyrest mit 1 bis 4 Kohlenstoffatomen ist;

$R^2$ ein Wasserstoffatom oder eine Methoxygruppe ist;

m den Wert 0 bis 2 hat;

n den Wert 2 bis 4 hat und $n^1$ den Wert 1 bis 4 hat.

2. Die D-Form einer Verbindung nach Anspruch 1 mit einem Substituenten R, der asymmetrisch ist.

3. 7 - [$\alpha$ - (Z) - (Methoxyimino) - 2 - furanacetamido] - 3 - [1 - [2 - [(carboxymethyl) - amino] - äthyl] - tetrazol - 5 - ylthiomethyl] - 3 - cephem - 4 - carbonsäure.

4. Ein Verfahren zur Herstellung einer Verbindung nach Anspruch 1, gekennzeichnet durch Umsetzen einer Verbindung der Formel II

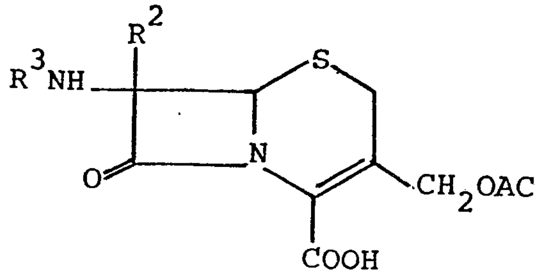

(II)

in der AC eine Acetylgruppe ist, oder ihrem Derivat, wobei die Cephem-4-carbonsäuregruppe geschützt ist, $R^3$ ein Wasserstoffatom oder ein wie vorstehend definierter Rest R ist, mit der Maßgabe, daß etwaige Amino-, Carboxy-, Sulfo- oder Hydroxygruppen gegebenenfalls geschützt sind, mit einer Verbindung der Formel III

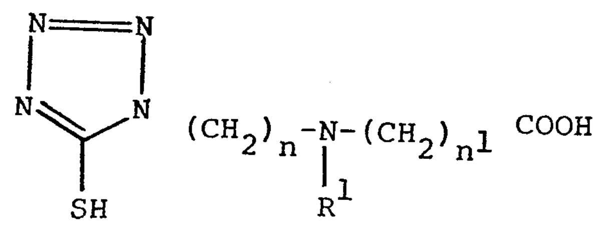

(III)

in der R', n und n' dieselbe Bedeutung haben wie in der Formel I oder ihrem Alkalimetallsalz, wobei, falls $R^3$ ein Wasserstoffatom ist, das erhaltene Produkt mit einem Acylierungsmittel oder einem aktivierten Derivat einer Säure ROH, in der R die in der Formel I angegebene Bedeutung hat, acyliert wird, mit der Maßgabe, daß etwaige freie Amino-, Carboxy-, Hydroxy- oder Sulfogruppen gegebenenfalls geschützt werden, und anschließend, falls $R^1$ ein Wasserstoffatom bedeutet, gegebenenfalls das Produkt mit einem N-Acylierungsmittel, das einen Niederalkanoylrest liefert, acyliert wird, anschließend etwaige Schutzgruppen entfernt werden und gegebenenfalls die so erhaltene Verbindung der Formel I in ein pharmazeutisch verträgliches Salz, einen Ester, einen leicht spaltbaren Äther oder ein Amidderivat überführt wird.

5. Eine pharmazeutische Zusammensetzung, enthaltend eine Verbindung nach einem der Ansprüche 1 bis 3 und einen pharmazeutisch verträglichen Träger.